Europäisches Patentamt

European Patent Office

Office européen des brevets

(19)

(11) Publication number: **0 319 272**
**A2**

# (12) EUROPEAN PATENT APPLICATION

(21) Application number: 88311347.4

(51) Int. Cl.⁴: **A61M 5/14**

(22) Date of filing: 30.11.88

(30) Priority: 04.12.87 US 128978

(43) Date of publication of application:
07.06.89 Bulletin 89/23

(84) Designated Contracting States:
DE FR GB IT NL SE

(71) Applicant: **PACESETTER INFUSION LTD.**
**trading as MINIMED TECHNOLOGIES**
**12884 Bradley Avenue**
**Sylmar California 91342(US)**

(72) Inventor: **Koenig, Paul A.**
**26058 Tampico Drive**
**Valencia California 91355(US)**
Inventor: **Hague, Clifford W.**
**27069 Crossglade Avenue No. 4**
**Canyon Country California 91351(US)**
Inventor: **Slate, John B.**
**4084 Kraft Avenue**
**Studio City California 91604(US)**
Inventor: **Colman, Fredric C.**
**2301 Roscomare Road No. 10**
**Bel Air California 90077(US)**
Inventor: **Rule, O. Rey, III**
**2221 Ocean Avenue**
**205 Santa Monica California 90405(US)**

(74) Representative: **Rees, David Christopher et al**
**Kilburn & Strode 30 John Street**
**London WC1N 2DD(GB)**

(54) User interface for multimode medication infusion system.

(57) A user interface for clinical configuration of a multimode medication infusion system for use with a disposable fluid pathway that incorporates a sterile cassette containing pumping elements and sensor interfaces in a multi-channel configuration. The hardware portion of the user interface comprises an audio signal generator (26), status indication devices (24), an information display (28) and user inputs (20). A programmed microprocessor (22) allows the user to control the system through the user inputs (20). The display (28) allows simultaneous display of data pertaining to multiple infusion requirements. In an instrument configuration mode, the interface communicates with a computer (12) running specialised software (16) to allow the changing of default values for various parameters of the system.

Fig.1

## USER INTERFACE FOR MULTIMODE MEDICATION INFUSION SYSTEM

The present invention relates generally to an electomechanical system for continuously infusing medication into a patient and, more particularly, to a user interface for the configuration of such a system for automatic operation in selected modes.

Until recently there were two major techniques available for delivering drugs to a patient when the drugs could not be administered orally. The first technique is to inject the drug into the patient with a syringe and needle, to deliver an appreciable does at relatively infrequent intervals. This technique is not always satisfactory, particularly when the drug being injected is potentially lethal, possibly has undesirable side effects when given in a large dosage, or must be delivered more or letter continuously to arrive at the desired therapeutic result. This technique leaves much to be desired. The risks of overdosage or harmful side effects may be reduced by giving smaller injections at more frequent intervals, but this is an inconvenient and not altogether satisfactory alternative.

The need for delivering a drug more or less continuously to achieve a desired therapeutic effect gives rise to the second technique, which involves a continuous delivery of medication to the patient, typically through an intravenous drip. Medication may also be administered using an intravenous system with an injection into a complicated and cumbersome interconnection of IV tubes, hoses, and other components. Drop counters are used to measure the amount of fluid delivered, and medications are often delivered in a large dose through injection into the IV lines, with the medication being somewhat diluted by the fluid.

A relatively recent alternative to these two techniques of administering medication to a patient is the medication infusion pump. A valuable and much needed development, the medication infusion pump can be used to administer drugs to a patient in small, carefully measured doses at frequent intervals, or with some devices slowly but uniterruptedly. A therapeutic regimen with an infusion pump cam be controlled electronically to administer precisely measure quantities of a drug at precisely planned intervals to to give a gradual infusion of medication into the patient. The infusion pump therefore represents a closer approximation to the natural maintenance of biochemical balances in the body because of its operation in a respective small dose mode.

Disposability is an important consideration in the design of medication in fusion systems. Those parts of the system through which medication is pumped must be sterile, so that in most applications some of the equipment is used and then discarded. The disposable parts may be replaced at regular intervals, typically on a daily basis. Disposability of the fluid pump portion of the infusion device is a highly desirable feature. It would be very convenient to design a fluid pump in the form of an attachable cassette of economical design which could easily be installed onto a main pumping unit. A cassette which uses a small number of parts, is easily mass producible., and is capable of delivering liquid medication or other therapeutic fluids with a high degree of precision is the subject of the present Applicants' co-pending application No.          , entitled "Disposable Cassette for a Medication Infusion System."., the contents of which are incorporated herein by reference.

The disposable cassette which is referred to above includes a fluid pump affording a high degree of accuracy in fluid delivery, with the degree of accuracy being maintained throughout the life of the product.

The cassette also provides means for conveniently and easily priming the pump, and includes a bubble trap to prevent the frequent shutdowns and alarms which are a problem with presently available pumps. The cassette also includes additional devices such as a pressure sensor and a bubble detector which in conventional medication infusion systems constitute separate assemblies.

A fluid monitoring and control system for use with disposable cassettes is needed to ensure accurate and safe delivery of therapeutic fluids. The design of such a system requires careful attention to the factors affecting the accuracy of fluid delivery, and instrument monitoring functions are necessary to ensure safe operation of the system.

There has been a long-felt but unresolved need for the development of a medication infusion management system that can be used for patient care in both hospitals and home health care applications. A desirable system would provide a reliable and improved product for current applications to encourage the use of new therapeutic techniques, reduce the cost of hospitalisation by improving care and decreasing labour and inventory costs, and would be versatile enough to allow intra-arterial and subcutaneous infusions. The primary requirements of such a system would be volumetric accuracy, state-of-the-art safety functions, and a capacity for independently controlling more than one pumping channel, each with a separate line to the patient.

Ideally the pump of the improved medication infusion system would be substantially smaller and lighter than current hospital pumps while at the same time incorporating multiple pumping channels. Moreover, it is desirable to be able to configure selected system parameters and to monitor displayed information related to the needs and performance of a given system, thereby optimising the operation of the system. Together with the possibility of extended battery-powered operation, these features may be incorporated in a device that it particularly well suited to ambulatory care, intensive care, emergency transport, emergency care, or operating theatre use, as needed.

A system with the capacity for multiple pumping channels, a variety of disposable configurations, a maintenance mode, and a library of software functions could combine the capabilities of several currently available devices into one single unit. For example, the need in a hospital for separate syringe pumps, PCA pumps, neonatal pumps, general purpose pumps, and computer communications pumps could be eliminated in favour of one system that could satisfy the requirements for all these devices on a selective basis.

According to the invention, there is provided a user interface for a multimode medication infusion system, characterised by audio generation means; a plurality of status indicators; an information display; a plurality of user inputs; and a microprocessor, operatively connected to, and controlling the audio generation means, the status indicators, and the information display through the user inputs and through a plurality of input signals from sensors in the infusion system. Preferably, the microprocessor is arranged to be connectable to a digital computer running specialised software which enables a user to change default values of the operating parameters of the system. The display may be a liquid crystal display.

Preferably, the display comprising a screen divided into the following areas: a status line preferably at the top of the screen; a softkey label area preferably at the bottom of the screen; a prompt line preferably adjacent the softkey label area; and an information area preferably between the status and prompt lines. Preferably, the microprocessor is preprogrammed to provide a plurality of "pages" for display on the information display, each "page" determining the function of a subset of the user inputs. In a preferred embodiment, the "pages" comprise: a standard "page" ; a clinical configuration "page"; an instrument configuration "page"; a maintenance "page"; a pump status "page"; and a total volume infused "page".

Preferably, the user inputs comprise a plurality of hardware switches. These may include:an "on/off" power switch; a "standard display" switch; a "start/stop" switch; a "select A" switch for selecting pump A; a "select B" switch for selecting pump B; a "select C" switch for selecting pump C; and a plurality of "softkey" functions that depend on a current operating state of the system and are shown on a "page" displayed on the information display means. There may also be a patient-controlled analgesia switch. Preferably, the "A" switch, "B" switch and "C" switch each have two status indication means associated with them, namely, an "alarm" LED and an "infusing" LED.

Thus, a user interface for clinical configuration of a multimode medication infusion system is provided which has the desirable characteristics listed above. The apparatus is designed for use with a disposable fluid pathway that incorporates a sterile cassette containing pumping elements and sensor interfaces in a multi-channel configuration. The hardware portion of the user interface preferably comprises an audio signal generator, status light-emitting diodes (LEDs), a liquid crystal display (LCD) and user inputs. A programmed microprocessor allows the user to control the system through the user inputs. The audio signal generator is used to attract the attention of the operator, the status LEDs allow the operator to make a quick visual check of the status of the instrument at a distance or in a darkened room, and the LCD presents all the detailed information about the system status and operation.

The preferred user interface is designed to be flexible to allow the system to be used by relatively untrained personnel without sacrificing the capability for use by better trained personnel to control the more complex infusion regimens which are possible with the system. Since the system has more than one pumping channel, the user interface allows simultaneous display of data pertaining to multiple infusion requirements. A variety of complex infusion regimens are possible on each pump, making the system potentially very complex, and for this reason the design of the user interface has been kept as simple and intuitive as possible.

While setting up an infusion regimen, the operator deals with only one pump at a time. When monitoring one or more regimens, the operator is able to view the most important information from each pump at a glance. Information is grouped in a clinically useful way and displayed on the LCD in specific formats referred to as "pages". Using the interface consists mainly of selecting the correct pages to view and, if necessary, changing information, responding to alarms, and so forth. The most significant information for each pump is displayed on the LCD in a format known as the "standard page". Basic infusion parameters such as infusion rate and volume remaining, as well as information about alarms and overall status can be viewed from this display. The standard page is the default display which appears initially without operator intervention.

In the preferred embodiment, the user interface imitates a single-pump infusion device for the purposes

3

of setting up an infusion regimen. Information relative to only one pump, the "selected" pump, is displayed at one time. The operator has the option of changing the "selected" pump at any time.

Dedicated controls are provided by the interface device for turning the instrument on and off, selecting a pump, starting and stopping an infusion regimen, and immediately reverting to the standard display. Four "softkeys" are provided for greater flexibility of control. The function of each softkey is dependent upon the current state of the system and which page is displayed on the LCD. Softkeys are ranked in importance and sets of softkey options are displayed. A "More Options" control allows the operator to switch from one set of options to another; this control does nothing other than to switch the softkey definitions. The "More Options" control has no effect on fluid delivery; it only affects the operator interface.

Another feature of the preferred user interface is the status line of the LCD, which gives a concise indication of the status of all pumps in operation, no matter what the rest of the display shows. Finally, a line of the LCD is reserved to display prompting information. Prompts are designed to give enough information so that an operator can safely set up and review infusion regimen information, start and stop infusions, turn the instrument on and off, and respond to alarms.

The LCD may be backlit for viewing when ambient light levels are low. Contrast of the LCD display is adjustable to one of eight preset values.

The status LEDs may consist of an "alarm" LED (red) and an "infuse" LED (Green) for each pump. The LEDs may be positioned in the corners of the pump select controls and are bright enough to be seen from a distance of 25 feet in daylight.

As described in the Applicants co-pending patent application (P14217EP), incorporated herein by reference, a clinical user can configure the medication infusion system to emulate a variety of different devices. Through the changing of rate and volume remaining ranges, as well as patient-side occlusion and air-in-line alarm sensitivities, the the following types of devices can be emulated: general-purpose, neonatal, flow controller, operating room, and home health care.

The invention may be carried into practice in various ways and one embodiment will now be described by way of example with reference to the accompanying drawings in which:

Figure 1 is a schematic block diagram of the user interface in relation to a multimode medical infusion system;

Figure 21 is a front view of the user interface hardware;

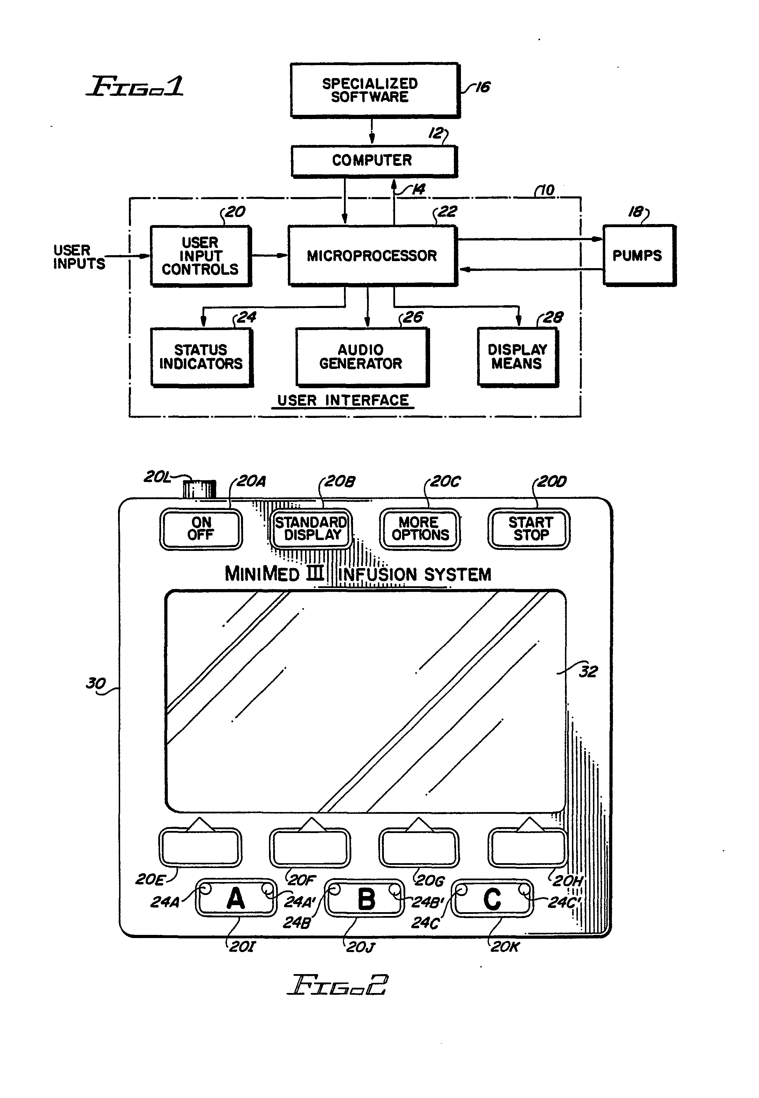

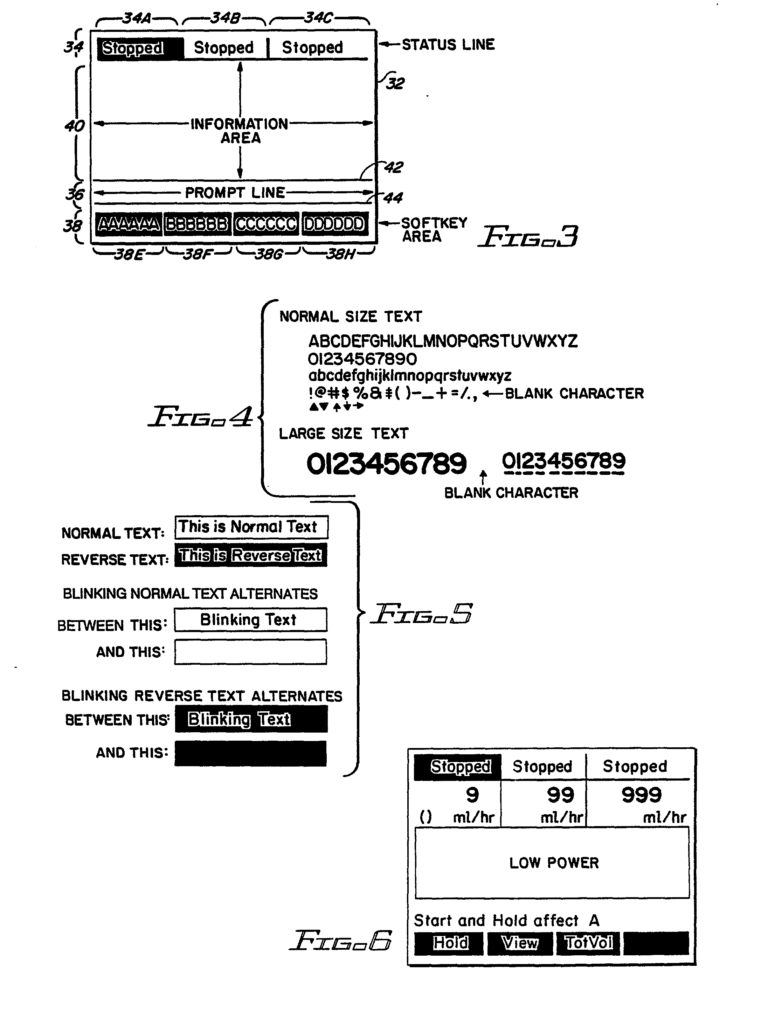

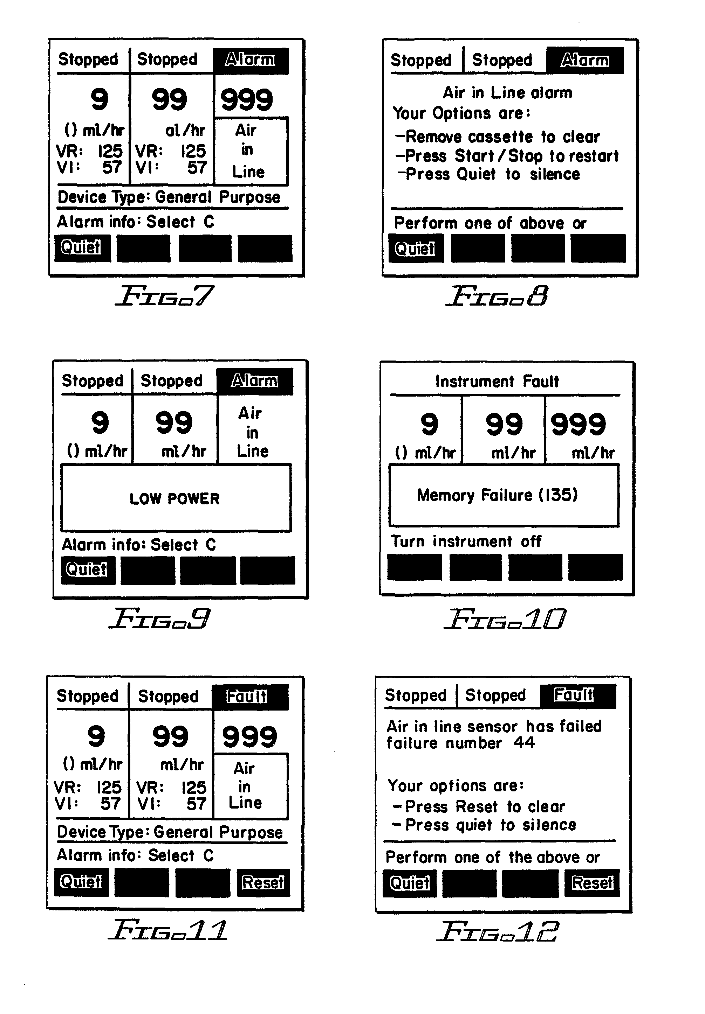

Figure 3 is a schematic diagram of the layout of the liquid crystal display;

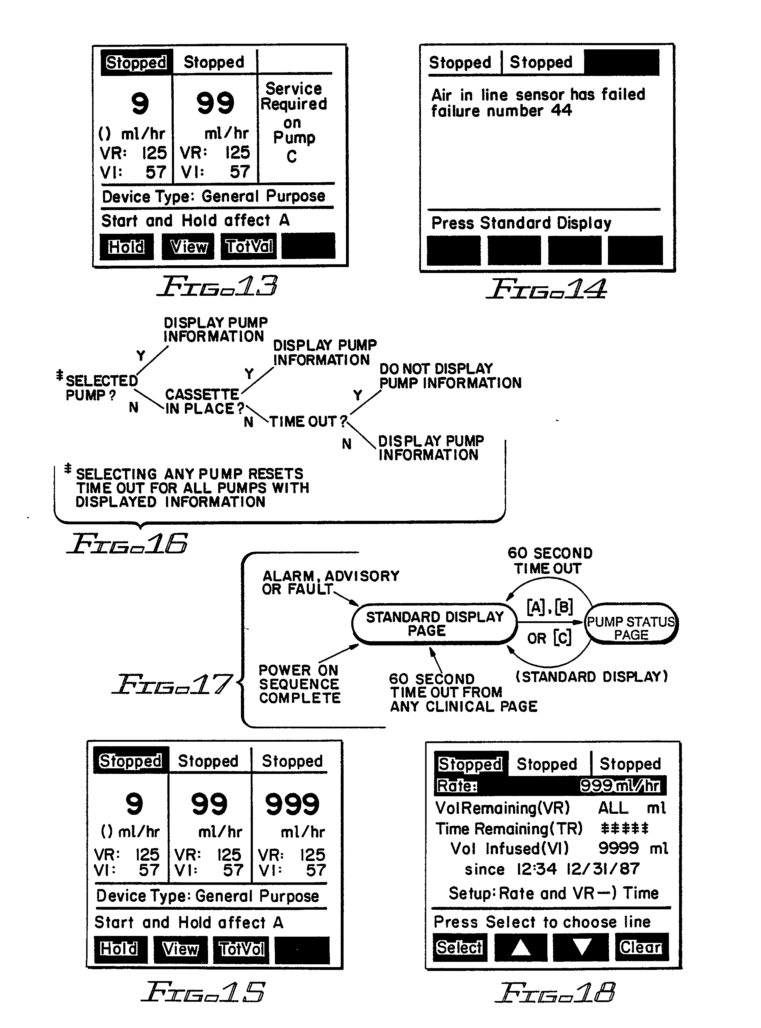

Figure 4 shows the font definitions for the liquid crystal display;

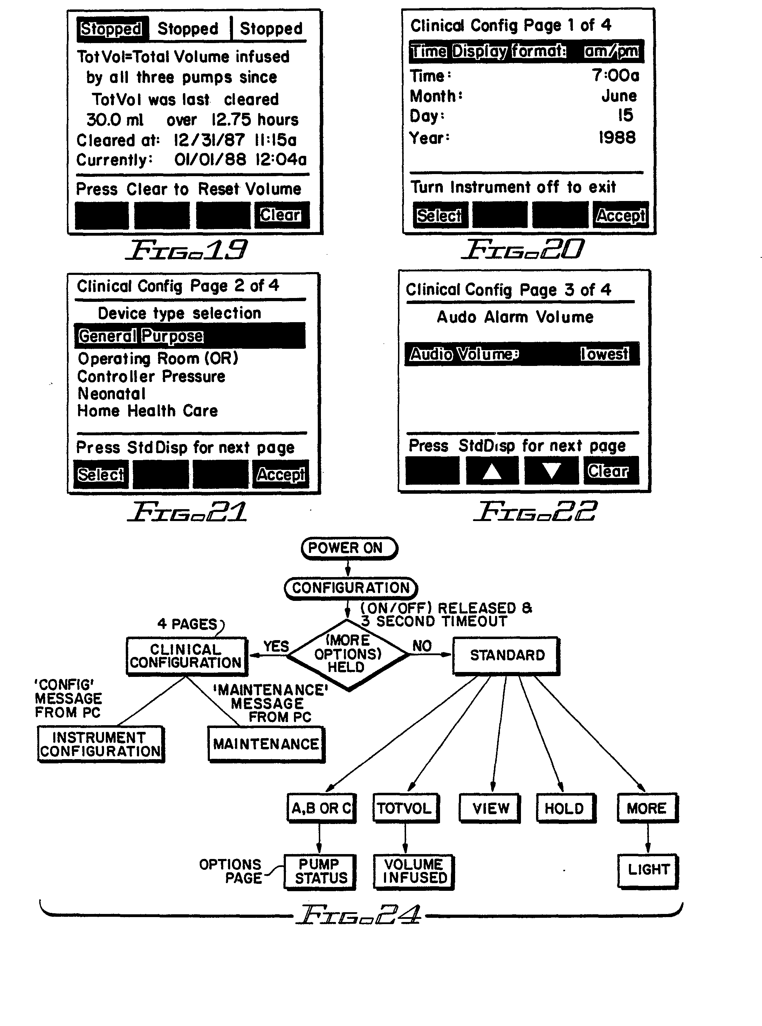

Figure 5 shows the text display attributes for the liquid crystal display;

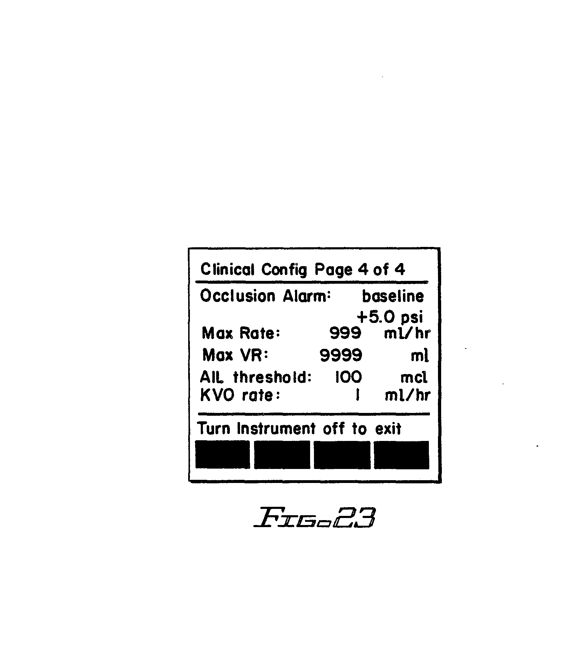

Figure 6 shows an example of an instrument alarm/advisory;

Figures 7 to 9 show examples of pump alarm/advisories;

Figure 10 shows an example of an instrument fault;

Figures 11 and 12 show examples of a pump fault;

Figures 13 and 14 show examples of inoperative pump indications;

Figure 15 shows an example of a standard page;

Figure 16 shows a decision tree for the display of pump information;

Figure 17 is a diagram of the timing for transitions between the standard page and the pump status page;

Figure 18 is an example of the volumetric pump status page;

Figure 19 is an example of the total volume infused page;

Figures 20 to 23 are examples of the clinical configuration pages; and

Figure 24 is a schematic flow diagram of the overall structure of the user interface.

Figure 1 is a schematic block diagram of a user interface for clinical configuration of a multimode medication infusion system as described in a number of related co-pending patent applications in the name of the present Applicants'. The user interface 10 is able to communicate with an off-line digital computer 12 via a communications interface 14. When the user interface 10 is connected to the computer 12 in this way, specialised software 16 is run on the computer 12 to enable selected qualified personnel to change default values for various parameters associated with operation of the medication infusion system. This mode of operation of the user interface 10 is called the "instrument configuration mode".

Normally, the user interface 10 is not connected to the computer 12, but controls the functioning of a medication infusion system employing a disposable fluid pathway that incorporates a sterile cassette containing pumping elements 18 and sensor interfaces in a multi channel configuration, as described in the present Applicants' co-pending application No. (P14217EP). The user interface 10 comprises user input controls 20, a microprocessor 22, status indicators 24, an audio generator 26, and display means 28.

As shown in Figure 2, the user interface 10 has four basic elements : an audio signal generator, status light-emitting diodes (LED), a liquid crystal display (LCD), and a plurality of user inputs. A user interface chassis 30 houses a liquid crystal display 32, above which are four user input controls 20a-20d, and below which are user input controls 20e-20K.

The input controls 20a-20d are momentary-contact switches labelled "on/off", "standard display", "More Options", and "start/stop", respectively. Switches 20e-20h are so-called softkeys, whose functions depend on what is being displayed on the LCD 32. The switches 20i-20k are used to select a pump for infusion. A switch 201 is a patient-controlled analgesia switch.

The face of each pump select switch contains two status LEDs. Thus, the pump select switch 20i has status LEDs 24a and 24a', the pump select switch 20j has status LEDs 24b and 24b', and pump select switch 20k has status LEDs 24c and 24c'. The status LEDs 24 allow the user to make a quick visual check of the status of the instrument from a distance or in a darkened room, and the LCD 32 presents all detailed information about instrument status and operation. The user inputs 20a-20k allow the operator to control instrument operation.

Normally a user will want to deal with only one pump at a time when setting up an infusion regimen. The user interface 10 is designed to facilitate this by grouping information in a clinically useful way on the LCD 32 in a specific format referred to a "page". Many different types of pages are defined for the instrument. Reference is made to the present Applicants' co-pending application No. (P14217EP) for specific details of the various pages which are available for display on the display device 32. However, details of the clinical configuration pages will be described hereinbelow.

Figure 23 indicates the overall operational structure of the user interface, and boxes with rounded corners denote liquid crystal display pages. The transitions from one LCD page to another are shown. The event which triggers a transition is shown in a rectangle superimposed on the transition (an operator activation of a control) or a lable next to the transition (an instrument-triggered change). All transitions operate from top to bottom or left to right. For example, to mover from the standard page to a pump status page, the operator activates a pump select key "A", "B" or "C".

Many pages have "More Options" softkey functions defined. Note that a "More Options" activation without any corresponding display change denotes that the primary set of softkey options is re-displayed. If no secondary sets of softkeys are defined, the "More Options" softkey has no effect.

All pages subordinate to the standard display have a transition to the standard display after 60 seconds of front panel keyboard inactivity. In addition, there is an implied transition from all LCD pages to the standard page, using the "standard display" key. An implied transition from all clinical operations display pages to a pump status page exists, by activation of the appropriate pump select key "A", "B" or "C".

Some boxes in Figure 23 show more than one softkey function. Only one of the functions in a box is available at any time, depending on conditions not shown on the chart.

The LCD 32 is used for all data entry and display for the system. Four types of information are presented:

    a) General status information for each pump:
    b) Prompts and other information to assist in stetting up and using the pump;
    c) Softkey labels; and
    d) Detailed information about the instrument status and status of each of the pumps.

As shown in Figure 3, in order to present this information sensibly, the LCD 32 is divided into four sections; a status line 34, a prompt line 36, a softkey area 38 and an information area 40.

The LCD 32 is used to display both general and detailed information about the status of the instrument, each of the pumps, and any infusion regimens. Most of this information consists of alphanumeric text. Certain other visual effects are used to enhance the readability of the display. Characters are displayed using two different fonts, as shown in Figure 4. Normal size text is made up of alphanumeric characters of five pixels in width by seven pixels in height. The characters are displayed in cells that are six pixels wide and ten pixels high. These characters are used for displaying the bulk of the information on the LCD 32, and are designed to be easily read from a distance of three feet.

Large size text is made up of characters twice as large as normal size text, i.e. each character is 14 pixels high by 10 pixels wide. These characters may be displayed on arbitrary pixel boundaries, that is, the position of these digits is not confined to specific cells. This character set is used to display data that must be viewed from a distance of eight to ten feet. This character set consists of the characters necessary to display numeric data only. Digits to the right of the decimal point are displayed using characters which are composed of slightly shorter, underlined digits, as shown in Figure 4.

Text is normally displayed as dark pixels on a light background, but can also be displayed as reverse-contrast text, as light pixels on a dark background. When displaying a block of text in reverse-contrast, the characters always have at least one row/column of background pixels surrounding the text so that the text does not bleed to the edge of the screen. When portions of the screen blink, they all blink in unison. Blinking areas alternate at 500 millisecond intervals between the normal contents of the block and a block of the background intensity, as shown in Figure 5.

Refererring again to Figure 3, the status line 34 of the LCD 32 is used to display the overall status of each pump. This area 34 is divided horizontally into three areas 34a, 34b, and 34c, each area corresponding to one of the pumps. Each of the areas 34a-34c is limited to eight characters of normal size text. The status of the selected pump is displayed in reverse contrast text. In Figure 3, for example, the pump "A" has been selected. The possible status values are:

| | |
|---|---|
| Fault | pump requires service |
| ALARM | alarm exists |
| Stopped | infusion regimen stopped |
| Infusion | fluid delivery in progress |
| Hold | operation is suspended |
| KVO | delivering at keep vein open rate. |

If the instrument is operating in a non-clinical mode, the status line 34 displays the operating mode as either "instrument configuration", "clinical config", or "maintenance".

The prompt line 36 is located just above the softkey area 38 and is separated from it and the information area 40 by horizontal lines 42 and 44. The prompt line 36 can display a maximum of 27 characters. The prompt line text display is dependent upon which page is active and what the sate of the instrument is. Prompt text is always displayed in normal size characters. Certain pumps may be in reverse contrast characters.

The softkey area 38 includes the bottom portion of LCD 32. Four separate areas 38e-38h make up the softkey area 38, with each area centred above a softkey. Four blocks of text can be displayed, with each softkey label displayed as six normal size reverse-contrast characters.

The main portion of the LCD 32 between the prompt line 36 and the status line 34 is the information area 40, which is used to display whatever information is relevant at any given time. Information area 40 can display six lines of 27 normal size text characters.

The LCD 32 requires backlighting when ambient light levels are low. The LCD backlighting turns on whenever any of the front panel controls are pressed. The backlighting remains on until the front panel controls have been inactive for a selected interval; e.g. 30 seconds. No direct means for turning the LCD backlight off is available. In addition, the LCD backlighting is activated when the instrument is turned on, and does not turn off until the keyboard has not been touched for 30 seconds. Finally, if the instrument is running off an AC adapter, the backlighting is activated when any alarm, advisory, fault, or prompt is given. The backlighting remains on as long as the advisory, alarm fault or prompt is preset.

The contrast of the LCD 32 may require a slight adjustment when instrument orientation is changed. The contrast is adjustable to one of the eight preset values using the softkey "view" available from standard page. When this softkey is activated, the viewing angle changes one increment every 240 milliseconds. When the viewing angle back to the other extreme, that is the viewing angle wraps around.

Whenever the instrument is used in a clinical operating mode, the standard display page is re-displayed if no front panel control is pressed for 60 seconds.

As shown in Figure 2, each pump has two LED indicators, a red "alarm" LED and a green "infuse" LED. The LEDs are positioned in the corners of the pump select controls 24a-24c with the "alarm" LED to the right and the "infuse" LED to the left. Thus, in Figure 2, LED 24a is a green "infuse" LED and LED 24a' is a red "alarm" LED. These LEDs are bright enough to be seen from a distance of 25 feet in daylight. All status LEDs 24 are on for 1000 millisecond during the instrument power-on procedure, to enable the operator to check the LEDs 24 for proper operation.

The "alarm" LED for a pump blinks whenever a pump alarm or pump fault occurs for that pump. An instrument alarm or fault causes all "alarm" LED's to blink. Note that advisories do not cause the "alarm" LSD's to blink. The "alarm LED blinks at a rate of 2Hz, with a 20% duty cycle (100 ms on phase followed by 400 ms off phase).

The "infuse" LED for a pump is used to indicate that the pump is delivering fluid. If the instrument is attached to an AC adaptor that is supplying power, the "infuse" LED is lit for each pump which is delivering fluid. If the instrument is operating with battery power, the "infuse" LED blinks at a rate of 1 Hz , with a

10% duty cycle (100ms on phase followed by 900 ms off phase).

The user inputs on interface 10 are the front panel controls 20a-20K and the patient controlled analgesia (PCA) demand switch 201. These controls include four softkeys 20e-20h and seven dedicated switches 20i-20k. Each softkey function is labelled on the LCD 32 screen, immediately above the softkey. The keys 20e-20h themselves are not labelled.

The "on/off" control 20a allows the user to power the instrument on and off. When the instrument is "off", activating this control supplies power to the instrument electronics and causes the instrument electronics to reset. When the instrument is "on" and operating normally, activation of control 20a is sensed and results in a controlled shutdown of the instrument, ending with removal of power from the instrument after switch 20a is released. When the instrument is "on" but a malfunction has occurred (i.e. a watchdog has fired), activation of key 20a immediately removes power from the instrument.

Each of the pumps 18 shown schematically in Figure 21 has a corresponding pump select key 24, as shown in Figure 2. Each key is situated to line up with the disposable cassette for its associated pump 18. The keys 24a-24c are labelled "A", "B" and "C". Activating a pump select key 24 makes the associated pump 18 the "selected" pump, and causes LCD 32 to change to the pump status page.

The "start/stop" key 20d is used to start and stop infusion regimens. Activating the key 20d toggles the infusion status of the selected pump.

The "More Options" key 20c is used to display more softkey functions for a particular page. when activated, this control effects the display of the next set of softkey functions currently available. If only one set of softkey functions is available, pressing this key has no effect. If the last set of functions is currently active, pressure on the key 20c activates the first set of functions. In addition, the "More Options" control 20c is used to enter the clinical configuration mode after the instrument has been turned on.

The "standard display" control 20b allows the user to return to the standard page.

The operation of the system will now be described.

Supplying Power to the System

Power to the system is supplied by operator activation of the on/off switch. Pressing this control while the instrument is "off" supplies power to the electronics (assuming that the internal batteries are charged or an external power supply is attached) and causes an instrument reset. The instrument then:

A) Performs a "power-on self test" (POST)

b) Displays the current instrument configuration;

c) Determines whether to operate in a non-clinical operating mode; and

d) If clinical operation is entered, the standard page is displayed. Otherwise, the first clinical configuration page is displayed.

Power-on Self Test

The purpose of the power-on self test is to ensure that the major instrument components are operating normally. The POST must be completed in less than three seconds, and therefore only part of the operation of the instrument is tested. A pump which is inoperative is still tested, and if it passes all tests, it is no longer marked as inoperative. If the instrument or any of the pumps fail a test, the test that was failed is stored, and the instrument continues with the remainder of the self-test procedures. At the end of POST if any faults have been found, an appropriate message is displayed on the LCD. If the fault is confined to one pump (a pump fault), the pump is marked as inoperative. IF the fault relates to the functioning of the instrument as a whole (an instrument fault), the operator must have the instrument serviced. If a pump fails a test for which it is marked as inoperative, no error message is given. However, if an inoperative pump fails a different test than the one for which it is marked inoperative, an error message is given.

Configuration Page

During the POST instrument displays the configuration page. The following information is presented:

a) Device type;

b) Software version number; and

c) Current date and time.

This page remains active until the "on/off" control is released, or for three seconds.

Entry Into Operation

When the operator releases the "on/off" control, the instrument determines which operating mode should be initiated. Normally the instrument enters clinical operation, but the clinical configuration mode may be entered by pressing he "more options" control before releasing "on/off". When the instrument enters clinical operation, a power-up OK audio signal is given.

Turning the Instrument Off

The instrument is turned off by the operator activating the "on/off" control while the instrument is "on". The following steps are taken.

a) Fluid delivery stops for all pumps;

b) Any memory updates are completed;

c) All cassette homing sequences in progress are completed

d) All audio signals are silenced, all LEDs are turned off, the LCD is blanked, and the backlighting is turned off; and

e) When "on/off" is released, power to the instrument is removed and the instrument shuts down.

Pump Selection

The operator can select any of the pumps by pressing the corresponding pump select control "A", "B" or "C". Activation of one of these controls also activates the corresponding pump status page. When power is turned on the most recently selected pump is selected. If no cassette is installed on the selected pump, the left most pump with a cassette installed is selected. Timing considerations and the relationship between the standard display and pump status pages are illustrated in Figures 16 and 17.

Infusion Regimen Setup/Review

The operator can review the status of the three pumps by using the standard page display, which is produced by pressing the "standard display"key. All pages reached from the standard page, either directly or indirectly, will be replaced by the standard page after a 60 second timeout. In formation relevant to the current infusion regimen setup is displayed for each pump

The following information is displayed for an RVT infusion:

Volumetric infusion rate (ml) is displayed in large digits. If the instrument is set up as a neonatal pump, the infusion rate display always includes one digit to the right of the decimal point. If the device is a home-health care type, a digit to the right of the decimal point is displayed if the rate if less than 100. Volume remaining (ml) is displayed in normal-sized digits. Volume infused (ml) is displayed in normal sized digits. The current device type is displayed. When an empty container detector is attached, an icon is displayed in the information area for each pump.

A "Tot Vol" softkey is available which provides access to total volume infused information. A "view" softkey changes LCD viewing angle. A "hold" softkey changes the status of the selected pump to "hold". This softkey is available only if the selected pump status is "stopped".

Two "more option" softkeys are available. A "light" softkey enables or disables backlighting in the home-health care mode.

When the instrument is turned on, the standard page presents the status for all pumps with a cassette in place. Putting in a cassette brings up information about the corresponding pump on the standard page, as well as restarting the 120 second timer for all pumps with information displayed. When a cassette is removed, information about that pump is displayed for 120 seconds and then disappears if the cassette has not been reinstalled. If another pump is selected, the information for the pump without a cassette is removed from the LCD.

8

Volumetric Pump Status Page

Access to this page is gained by pressing a pump select control. The information displayed is volumetric infusion rate (ml/hr), volume remaining (ml), time remaining (hours: minutes), volume infused (ml) date and time at which volume infused was cleared, and infusion type (V/R or V/t).

Data entry softkeys are available. A "select" softkey is used to select a parameter to change. Volumetric infusion rate, volume remaining, time remaining, volume infused, and infusion type may be selected. Two other softkeys are used to alter parameter values, volumetric infusion rate, volume remaining, time remaining, and infusion type. A "clear" softkey is used to set the selected parameter to a particular value:

| Infusion | : | KVO rate |
| Volume Remaining | : | All ml or 1 ml depending on infusion type |
| Time Remaining | : | 0 hours, 1 minute |
| Volume Infused | : | 0 ml |

The "recall" key retrieves the current value of the parameter before the value was changed using the "clear" or the changes of keys. Pressing the "accept" key enters the newly entered value for the selected parameter.

Setting Rate, Volume Remaining and Time Remaining

The minimum value that the operator can actually set for an infusion rate is the currently set KVO rate for the instrument. Also, the maximum infusion rate and volume remaining may be limited in the instrument configuration mode. Infusion regimens are commonly specified as follows:

a) The fluid is to be delivered at a specified rate. No end point for the regimen is given, i.e. no volume of fluid or infusion period, is specified.

b) A volume of fluid is to be given at a specified rate,

c) A volume of fluid is to be given over a specified length of time.

d) All the fluid in a container is to be delivered at a specified rate. ("infuse "all regimen).

To accommodate these methods of specifying an infusion regimen, and to minimise the probability of error when the operator enters the required parameters, an infusion type is set for each pump. The infusion type is either volume/rate or volume/time.

During a volume/rate type of infusion, the operator may set only the volume remaining and the infusion rate. Accepting a new value for rate or volume remaining causes the time remaining to be recalculated and displayed. If volume rate equals "all", the time remaining is indeterminate and is displayed as "----". If the current values of infusion rate and volume remaining specify a time remaining less than the minimal time remaining allowed, the time remaining is prefixed with the character "<". For example:

| Infusion Rate | : | 100 ml/hr |
| Volume Remaining | : | 1 ml |
| Time Remaining | : | < 00h 01m |

If infusion rate and volume remaining are entered in such a combination that the calculated time remaining is greater than the maximum time remaining, the time remaining is prefixed with the character ">". For example:

| Infusion Rate | : | 1 ml/hr |
| Volume Remaining | : | 250 ml |
| Time Remaining | : | > 99h 59m |

During a volume/time type of infusion, the operator may set only the volume remaining and the time remaining. Accepting a new value for either volume remaining (VR) or time remaining (TR) causes the

9

infusion rate to be recalculated and displayed. IF the operator enters a combination of VR ad TR that specifies a rate that is out-of-range, a prompt is given when the operator attempts to accept the new value.

If the operator selects the dependent infusion parameter (i.e.time during a volume/rate infusion or rate during a volume/time infusion), the promptline instructs the operator that the configuration must be changed to change the selected parameter.

The operator changes between the volume/rate and volume/time infusion types using the "setup" line on the pump status page. If the selected pump is currently delivering fluid this line may be selected , but the infusion type may not be changed. When the infusion type is changed, the rate volume and time are set to preset values:

| Volume/rate VR | = | All |
| Rate | = | minimum infusion rate |
| Time | = | indeterminate (displayed as "-------") |
| Volume/rate VR | = | minimum valid VR |
| Rate | = | minimum valid rate |
| Time | = | calculated from Vr, Tr. |

## Total Volume Infused Page

The total volume infused page is accessed by pressing the "Totvol" control on the standard page. The total volume infused since a given time is displayed in millilitres, with resolution of 0.1 ml. This parameter is a separate running total of all fluid delivered by all pumps, and does not necessarily equal the algebraic sum of the volume infused for each pump. The number of hours between the time at which the total volume infused was last cleared and the current time is also displayed. This value has a 0.05 hours resolution. If the value is outside the range of 1 minute to 99h 59m or if TVI equals 0.0, it is displayed as "------". The time and date at which total volume infused was cleared is also displayed, as well as the current date and time.

A "clear" softkey is used to clear total volume infused displayed. An "accept" softkey is used to reset the TVI stored to 0.0ml. See Figure 19 for an example of the total volume infused page.

## Starting/Stopping Infusion Regimens

The "start/stop" control is used to activate and suspend infusion regimens. This control directly affects only the selected pump. The exact response of the instrument to activation of this control, is dependent on the status of the select pump:

a) Stopped--Pressing "start/stop" activates the infusion regimen on selected pump.

b) Infusion--pressing "start/stop" suspends fluid deliver.

c) ALARM--if a pump alarm is present on the selected pump, pressing "start/stop" usually clears the alarm and resumes fluid delivery.

d) Hold--pressing "start/stop" activates the infusion regimen on the selected pump.

e) DVO--"start/stop" stops fluid delivery and changes status to "stopped".

If the currently selected pump is inoperative, "start/stop" has no effect.

## Infusion Hold

Normally, if any pump has a cassette installed, but is left unattended with no infusion regimen active, an operator callback advisory is given. However, there are situations in which it is useful to have a pump set up, with a cassette in place, but an operator callback is not required. Infusion hold is used by the operator to disable the operator callback advisory for a pump. The operator presses the "hold" softkey available from the standard page, to put the selected pump on hold. "Hold" is only available if the selected pump has a status of "stopped". The status for the pump becomes "hold". This mode is exited by pressing the pump select control for the pump (status becomes "stopped"), by starting an infusion regimen on the pump using "start/stop" or by removing the cassette (status becomes "no set").

## All Mode and Fluid Detector

The operator has the option of specifying that all of the fluid in a container is to be delivered to the patient. This is done by scrolling the volume remaining value until the value "ALL" appears. The value "ALL" may be set only if the selected pump is set up for a volume/rate type infusion. The value "ALL" appears in place of a VR value of zero. When VR is set to "ALL", the time remaining is indeterminate.

The "ALL" mode is available whether or not a fluid detector is attached to the pump. If a fluid detector is attached, the instrument delivers fluid until an empty container is detected; the instrument then issues an "infusion complete" advisory, and the pump changes its delivery rate to the DVO rate. If no fluid detector is attached, the instrument delivers fluid until the air-in-line detector detects air and an air-in-line alarm is driven, stopping fluid delivery by that pump.

When a fluid detector is attached to the instrument, the instrument gives an audio signal as operator feedback. In addition an icon is displayed on the standard page for each fluid detector which is attached to the instrument. The icon is displayed in each pump information area, on the left end of the third text line. If the fluid detector is removed from a pump while an infusion regimen is in progress for that pump, an alarm is given.

## Home-Health Care Instrument

When the pump is configured as a home-health care instrument, certain functions of the instrument are altered to prevent accidental control activation and to maximise the operational life of the battery packs. The instrument operates in low-power mode. When the instrument is "on", the "on/off" and "start/stop" controls must be held down for one second before the instrument powers down or the infusion regimen starts or stops. A general feedback signal is given by the instrument. If the control is released in less than one second, the control activation is ignored.

## Non-Clinical Operation

Because the instrument is capable of operating in a wide range of environments, performing extremely sophisticated functions, it is necessary to configure the operation of the instrument to the environment to which it is to be used. Without this configuration ability the user interface would become much more complicated. In addition, it is necessary to be able to test and maintain the operation of the instrument.

Configurability and maintenance functions must be performed when the instrument is not being used to infuse fluids into a patient.

Therefore, these functions are not available during normal operation and require special procedures in order to be accessed.

Configuration procedures are of two types: instrument configuration and clinical configuration. The basis for this division is the level of security required for the two configuration modes. Instrument configuration involves changing fairly sensitive information in the instrument, and is expected to be performed only in the biomedical engineering departments. The setting done in this mode are not be changed by clinical personnel. Clinical configuration mode covers those parameters that may be changed by a knowledgeable clinical operator, based on the requirements of the patient and the environment. Maintenance functions should be confined to the biomedical engineering departments. To ensure that maintenance and instrument configuration functions are only performed outside of the clinical environment, these functions can be accessed only by using the communications capability of the instrument.

## Clinical Configuration Page

The clinical configuration page is accessed by holding the "more options" key before releasing the "on/off" control at instrument power on. Page 1 displays a time display format which includes time, month, day, and year. Page 2 displays the device type, Page 3 displays audio alarm volume, and Page 4 displays the current values of parameters which are determined by the device type.

Softkeys are available for data entry. "More Options" softkeys are available as needed for data entry. The operator may toggle between the three pages by pressing "standard display". The only exit from clinical configuration is to turn off the instrument using "on/off". See Figures 20, 21, 22 and 23 for examples

of the clinical configuration pages.

## Instrument Configuration Modes

The instrument configuration mode allows the knowledgeable biomedical engineer to customise the instrument for a particular environment. The engineer has the ability to change the default values for various parameters normally determined by the current setting of the instrument type. Access to this mode is limited to those people with access to software written to operator on an IBM PC which interfaces to the instrument and implements the protocol necessary to operate the instrument in this mode.

The instrument configuration mode requires that the instrument first be placed into clinical configuration mode and then communications between the pump and the PC running the configuration software is initiated. The PC transmits a message to the instrument which places the instrument in instrument configuration mode. When the instrument configuration mode is entered, the instrument configuration mode page is displayed on the liquid crystal display.

The instrument configuration page is accessed by reception of a correct massage through an RS -232-C communications port, while in clinical configuration mode. The information "instrument configuration " is displayed on the status line. No softkeys are available. The only exit is by turning the instrument off using "on/off".

The operator can change settings for each of the five device types (OR, GP, controller, neonatal, HHC). These settings replace the default settings set up by the instrument initialisation. The biomedical engineer can alter the following parameters in the instrument configuration mode:
   a) Occlusion detection pressure;
   b) Air-in-line sensitivity;
   c) KVO rate; and
   d) Infusion rate and volume remaining limits.

## Maintenance Mode

The maintenance mode of the instrument is provided to allow the biomedical engineering department to repair easily and initialise the instrument. Like instrument configuration, it is only available over the communications link.

The maintenance mode page is accessed by reception of a correct message through an RS-232-C communications link while in clinical configuration mode. The information "maintenance mode" is displayed on the status line. There are no softkeys available. The only exit is by turning the instrument off, using the "on/off".

## Error Log

The operator may:
   a) Download the error log from the instrument;
   b) Clear the error log

## Instrument Initialisation

The operator may initialise the instrument to factory settings. Since this may involve changes in the communications parameters, the operator must be prepared to re-establish communications between the PC and the instrument. The following levels of initialisation are available:

Infusion data return

All infusion data for all pumps is set as follows:

| Rate | 1 ml/hr |
|------|---------|
| VR | All |
| TR | Intermediate |
| VI | 0.0 |
| VI | cleared none |
| TVI | 0.0 |
| TVI | cleared none |

Clinical configuration

Device type set to general purpose
Time set to midnight
Date set to 1/1/87
Time display format set to AM/PM
Audio volume set to medium

Maintenance data

Error log initialised
Calibration data cleared
Communication data initialised

Instrument configuration parameters

All data for all five device types set to default values.

Instrument Calibration

All instrument calibration procedures are performed in maintenance mode. Details of those operations require information about the sensors.

Data Entry

When entering or changing data on the instrument, the user performs three tasks:
a) Selecting the parameter to be altered (e.g. Infusion rate). This may require changing the selected pump;
b) Changing the value of the parameter (e.g. changing infusion rate from 100ml/hr to 125 ml/hr); and
c) Instructing the instrument to implement the new parameter value (e.g. "accept" or "enter" the value).
In addition, a means is always available to allow the operator to cancel a change made in a parameter value before the change is implemented. There are three types of data which are required:
a) Numeric (e.g. entering infusion rate);
b) Alphanumeric (e.g. entering patient ID information; and
c) Selecting a value from a list (e.g. choosing a drug from a list of drugs for use with the drug calculator).
The following sections describe the means by which the operator can select, change, and accept parameter values for each of these types of data.

Numeric Data Entry

To select a parameter to change, the operator activates the page that contains the parameters that are to be changed. Parameters are grouped together by function and importance; a page does not contain unrelated parameters. The user then moves the cursor on the LCD to highlight the parameter to be changed. Whenever a page that allows data entry is displayed, one of th parameters on the page is displayed in reverse-contrast test (highlighted). The operator moves this cursor using the "select" softkey. This softkey highlights the next parameter, i.e. the cursor moves to the newly selected parameter. Pressing the "select" key when the last parameter on the page is selected moves the cursor to the first parameter. Not all parameters displayed on the page may be selected; some parameters may require special actions by the operator before they can be changed.

Entry of numeric data is performed by scrolling the value of the selected parameter. When a value is to be changed, tow of the softkeys function to increment and decrement the value: ▲ and ▼ . Activating either ▲ or ▼ causes the number to begin to increase or decrease. If ▲ or ▼ is held down, the value increases or decreases at an accelerating rate. Since the range of values that parameters take is quite varied, the manner in which this acceleration occurs can be defined for each parameter. For example, when changing infusion rate, the value may initially change one unit per second for four seconds, then change at two units per second for four seconds, then eight units per second for two seconds, etc. When changing volume remaining, the value may initially change one unit per second for four seconds, then change at four units per second for four seconds, the sixteen units per second for two seconds, etc. The value stops changing when the softkey is released. If the softkey is then pressed again, the scrolling rate continues at the initial rate, not at the rate in effect when the softkey was released.

Once a value has been changed, "select" is no longer available. Therefore a new parameter cannot be selected until the new value for the selected parameter has bene cancelled or accepted. Another softkey, "clear", is used to reset the value to a preset value.

Whenever the value of the parameter is changed by pressing ▲, ▼ or "clear", two new softkeys are defined: "select" is replaced by "accept" and "clear" is replaced by "recall". Activating "accept" causes the changed value currently displayed to replace the value stored in the instrument with that value. The instrument then performs whatever actions are required to effect the value change. Activating "recall" causes the current value (the value before ▲, ▼ or "clear" was pressed) of a selected parameter to be displayed. Activating either "accept" or "recall" removes the "accept" and "recall" softkey options and re-enables the "select" softkey.

When a value is changed using ▲ or ▼ , the changed value blinks (when it is not scrolling). This alerts the operator that a value has been changed but not accepted.

When changing numeric parameters, there are often other parameters that are dependent on the value of the parameter being changed (e.g. time remaining is dependent on infusion rate). When dependent parameters exist, they are always displayed on the same page as the independent parameters.

## Alphanumeric Data Entry

Alphanumeric data entry operates in the same manner as numeric data entry, with some exceptions. Rather than changing one value, as with numeric data entry, the operator has the ability to change each character in the alphenumeric field separately. An alphanumeric field is selected using a specific softkey function. The parameter line is highlighted as before, but the character to be changed is displayed as normal text. The operator may then choose different character to be changed, using softkey ▲. When the last character in the field is selected, pressing ▲ selects the first character in the filed.

Each character is altered using the softkeys ⌃ and ~. Pressing these controls causes the character to change to the next or previous character defined in the font. For example, if the character "A" is pressed, the value will change from "C" to "B" to "A", etc. Figure 4 gives the definition of the characters in each font.

The "accept" softkey replace the "select" softkey whenever the alphanumeric value is changed using either ⌃ or ~. Activating "accept" enters the new alphanumeric value. A "clear" more option softkey clears the alphanumeric value to blanks. A "recall" more option softkey is available.

## Selection from a List

Most parameters on the instrument have a very small number of possible values. For example, time display format is limited to "am/pm" or "24 hr" formats. To enter or change these values, the operator is allowed to choose one of the values from a list of possibilities. Two methods for entering values from a list are required: scrolling through a list and selection from a list.

## Scrolling Through a List

When the list of possible values is shown, and space on the LCD is limited, only one value from the list is displayed at each moment. The parameter is chosen as with numeric data entry, using "select". The operator may display the next choice by pressing ▲, or the previous value by pressing ▼ . The operator may set the value to a preset base value by using "clear". The operator scrolls through the possible values, and then uses "accept" to enter the new value. "Recall" is available to retrieve the original value. The changed value blinks until it is either accepted or refused.

## Selecting from a List

The parameter to be altered is selected through a specific softkey function. If the list of possible values is too large to display on one page, the softkey function "page ▲" is used to bring up the next section of the list of values, and "page ▼ " is used to bring up the previous section. The action of these two softkeys will wrap around when the end of the list is encountered. The last value in a list which extends up to more than one page is followed by the text "end of list". The operator may not move the cursor onto this text. Once the proper selection of the list has been displayed, the operator can change the parameter value using "select". This softkey moves the cursor to the correct value on the page, wrapping around from the last to the first value on th page.

When the proper value has been selected, the operator must then accept the new value by using "accept". Once the cursor has been moved to a new value in the list, the highlighted value will blink until the new value is accepted or cancelled.

For example, to change the device type, the operator must enter clinical configuration mode and then activate the page which displays the device type. The desired device type is highlighted by moving the cursor with "select". Finally, the new device type is stored using "accept". The "select" softkey used during numerical data entry should not be confused witht he "select" softkey used to choose a value from a list. The numerical data entry "select" softkey allows the operator to select the parameter, and then ▲ and ◀ are sued to change the value. The later "select" function is used to choose a value from a list of possibilities. The parameter to be changed has already been determined.

## Audio Signals

A limited number of different audio signals are produced, each signifying a general condition that must be brought to the attention of the operator. Table 1 lists the different types of audio signals. Only one audio signal is used to indicate that an alarm condition exists. A different audio signal is used to indicate that an advisory exists or to give feedback to the operator when the controls are activated, etc. The two main functions of the audio generator have different requirements for audio volume and frequency range. The feedback function requires only an audio generator that can produce relatively low-volume sounds at one frequency. The requirement of being able to alert the operator requires an audio signal of much higher intensity, and a range of frequencies so that alarms can be distinguished from advisories, prompts, etc..

## TABLE 1

|  | Importance | Volume |
|---|---|---|
| Keyclick | Low | Level 1 |
| Power-up-OK | Low | Level 1 |
| Prompt | Low | Level 1 |
| Advisory | Medium | Variable |
| Alarm | High | Variable |
| Fault | High | Level 4 |
| Failure | High | Level 4 |
| General feedback | Low | Level 1 |

### Audio Volume

The audio generator is able to generate signals of four different intensities. The lowest intensity level (level 1) is easily heard by an operator operating the instrument in an intensive care unit environment. The highest intensity level (level 4) signal is audible at a distance of 15 feet in an intensive care unit environment. Levels 2 and 3 are set at uniform intervals between levels 1 and 4. Low-importance signals are always at level 1. Any signal indicating an instrument failure or fault is at level 4.

Medium- and high-importance signals begin at a volume level that can be selected by the operator. Every 60 seconds the volume of the signal is increased by one level up to level 4, until the signal is silenced or suppressed. The initial volume level is adjusted by the operator in the clinical configuration mode. If a suppressed signal is re-enabled, the audio volume is reset to the initial audio volume; it does not resume at the audio volume in use when the signal was suppressed.

### Audio Frequency

The audio generator produces a range of frequencies for 100 to 400, Hertz, with a resolution of 1/10 of an octave.

### Audio Signals

The keyclick signal is used to give feedback to the operator that a control has been pressed. The keyclick is always given when a control is pressed, unless the control is a softkey which has no function.

The general feed back signal informs the operator that either a fluid detector, a cassette, or a power pack has been properly connected to the instrument. It is also used to indicate that while the instrument is configured as a home-health care instrument, "on/off" and "start/stop" must be held for one second in order for these controls to have any effect.

The alarm signal indicates that one or more alarm conditions exist on the instrument.

The advisory signal indicates that one or more advisory conditions exist on the instrument.

The prompt signal is an indication to the operator that prompting information is momentarily being presented on the LCD.

The fault signal indicates that instrument software has detected a fault in the instrument which may affect proper instrument operation.

The failure signal is an indication that the instrument is not operating correctly.

The power-up-ok signal indicates that the instrument has passed the power-up self test and is ready for clinical operation.

It is possible that more than one of the audio signals may need to be produced at a particular moment.

For instance, it is possible that alarm, advisory, prompt, fault, keyclick, and dose demand signals could be presented simultaneously. Only the most important signal can be given at any one time. The following priority list exists, starting with highest priority: failure, keyclick, general feedback, fault alarm, advisory, prompt, and power-up-OK. An overridden audio signal is re-enabled as soon as the overriding signal is completed.

Alarms, Advisories, Prompts, Faults and Failures

Failures alert the operator to a malfunction resulting in performance that is out of specification, uncontrolled, or potentially dangerous to the patient. Failures are detected by the watch dogtimer. The only operator response to a failure is to shut the instrument off and have it serviced.

Faults, are malfunctions detected by the instrument software that could lead to performance that is out of specification, uncontrolled, or potentially dangerous to the patient. Faults make the instrument or one of the prompts inoperative until servicing is performed.

Alarms signal a clinical condition requiring immediate attention, but do not necessarily indicate that the instrument is defective. Alarm conditions cause fluid delivery to be halted.

Advisories denote a condition of which the operator should be made aware, but do not require immediate attention. Typically an advisory indicates that an action such as an infusion regimen has been completed. Advisories do not cause fluid delivery to be halted.

Prompts are used to guide the operator in the proper use of the instrument and are intended to informative without being offensive.

Silencing Audio Alarm/Advisory Signals

The audio signal can be stopped for a period of time without suppressing visual indicators and without restarting the infusion regimens. A suppressed audio signal is re-enabled after a short period of time which depends on how many alarms/advisories are present. The silencing interval ranges from 120 seconds for one alarm/advisory to 180 seconds for three or more alarm/advisories being present. Prompts cannot be silenced.

Clearing an Alarm/Advisory

The operator removes the cause of the alarm/advisory and then (in the case of an alarm) presses a softkey to inform the instrument to remove the alarm indicators and restart the affected infusion regimens. Advisories may be cleared by just removing the cause of the advisory; the operator is not required to activate any instrument control. Faults and failures cannot be cleared. There are times when the operator just wants to stop using the pump when a fault, alarm, or advisory occurs. Stopping a fault, alarm, or advisory removes the visual and audio indicators, but does not restart any affected infusion regimens.

Alarms

Two types of alarms exist: instrument alarms and pump alarms. Instrument alarms are those that affect the functioning of the instrument as a whole (e.g. low power alarm, locked off activation). Pump alarms are those alarms that only affect the pumps involved in an infusion regimen. If an infusion regimen involved more than one pump, an alarm on one pump can cause the entire infusion regimen to halt.

When any alarm is given, the standard page is activated and a window on the LCD displays a message briefly explaining the nature of the alarm. If the alarm is an instrument alarm, the window overlays the bottom half of the entire information area. A pump alarm window is displayed on the part of the information area for the affected pump. If no instrument window is present, the bottom half of the pump information area is used, otherwise the top half of the pump information area is used. (See Figures 7 to 9).

Instrument Alarms

See Figure 6 for an example of an instrument alarm. The selected pump does not change. All the alarm LEDs blink. A softkey function "quiet" silences the audio alarm signal. Instrument alarms are cleared by removing the source of the alarm and pressing the "resume" softkey. This restarts any infusion regimens that were halted by the instrument alarm. Instrument alarms are stopped by turning the instrument off.

## Pump Alarms

See Figures 7 to 9 for examples of pump alarms. The pump with the alarm becomes the selected pump. The alarm LED for the pump causing the alarm blinks. The "quiet" softkey silences the audio signal. In general, pump alarms are cleared by removing the source of the alarm and restarting the infusion "start/stop". This clears the alarm on the selected pump only. A pump alarm can be stopped by removing the cassette from the pump for which the alarm is given. If more than one pump alarm exists, the first pump with an alarm detected becomes the selected pump. All pump alarm windows are displayed simultaneously on the standard page and the operator can attend to whichever event is most serious.

## Advisories

When an advisory is issued, the effect on the user interface is similar to that of an alarm. An audio signal is generated and the LCD page may change. As with alarms, there are two categories of Advisories: instrument and pump.

If an alarm condition occurs while an advisory is in affect, the alarm has precedence over the advisory. The alarm condition must first be cleared and then the advisory continues. Advisories can be cleared by removing the cause of the advisory. The operator does not have to activate any any controls. For example, the low power advisory is cleared if the operator attaches a charged battery pack or an AC adaptor connected to AC power.

The LCD indicators for advisories are displayed in windows on the standard page, just as are the alarms (see Figures 6 to 9). Advisories do not cause any change in the alarm LEDs.

## Instrument Advisories

See Figure 6. The selected pump does not change. Instrument advisories are silenced using the "quiet" softkey. Instrument advisories ar stopped by turning the instrument off.

## Pump Advisories

The pump with the advisory becomes the selected pump. Pump advisories are silenced by pressing "quiet". If more than one pump has an advisory, the first pump with an advisory detected becomes the selected pump. Visual indicators for all pumps with advisories are presented on the standard page. The operator can attend to whichever event is most serious.

## Prompts

Prompts are brief instructions advising the operator which action to take next in order to correctly operate the instrument. Since it is impossible to be able to describe completely the proper operation of the instrument, only the most common operation will be described. Some general rules are that:

a) A prompt always relates to the condition of the selected pump.

b) When an alarm, fault, or advisory exists, the operator is prompted how to respond.

Some prompts persist for only a short time. Such a prompt is given in response to an invalid operator action such as attempting to start an infusion on a pump with no set installed. When a short-lived prompt is given, an audio signal accompanies it to help to alert the operator.

## Faults

At some point a fault in the instrument may be detected by the instrument software. A particular fault may not affect the overall instrument performance sufficiently to cause the watchdog timer to time out. Such faults are of two types; those that affect the operation of the entire instrument (instrument fault), and faults that affect only the operation of a single pump (pump faults). Either type of fault is logged in the instrument error log.

## Instrument faults

If the software detects a fault which does not allow any of the pumps to operate correctly , the fault is considered to be an instrument fault and the instrument must be shut down and serviced. The software performs the action required to stop infusions safely, and issues an indication of the fault. This failure indication consists of an audio signal that cannot be silenced, the blinking of all alarm LED's and description of the failure on the LCD (see Figure 10). Since an instrument fault may prevent some of the indications from being given (e.g. the LCD may be faulty), the full effect of the fault indications may not be noticeable.

The information presented on the LCD consists of a short description of the instrument fault as well as a reference number. The reference number is the same number stored in the error log and used in an instrument repair manual. The only possible action for the operator is to turn the instrument off, using "on/off". Any other control will be ignored.

## Pump Faults

If a fault is detected that affects the operation of only one pump, a pump fault is given. A fault indication consists of a blinking alarm LCD for the affected pump, an audio signal, and a description of the fault (see Figures 11 and 12). The operator must then stop the fault using "quiet"; it cannot be silenced or cleared. The pump cannot be used until the fault is corrected. The standard page indicates that the affected pump is inoperative (see Figure 13). If the associated pump select key is pressed, a pump status page with more detailed information about the fault is activated (see Figure 14).

## Watchdog-Detected Failures

The watchdog is the last line of defence against instrument failure. There may be some faults which the software is unable to detect or that may exist i the software itself. The watchdog is expected to prevent such faults from resulting in performance that is out of specification, uncontrolled, or potentially dangerous to the patient.

If the watchdog times out, the electronics of the instrument are held in a reset state, preventing the processor from operating and the pumping mechanism from delivering fluid. Since the processor does not operate, it is impractical to attempt to use the LCD or LEDs, and therefore the LCD and LEDs are in an indeterminate state. The audio signal generator is tied into the watchdog so that if the watchdog fires, an audio signal is generated.

The operator can only turn the instrument off at this point. The "on/off" switch is handled in hardware, as the microprocessor is disabled.

## Data Logging

The user interface has the capability of keeping a log of information for clinical and maintenance purposes. The charting log contains clinical information related to infusion regimens. The error log is a journal of all errors in the instrument detected by the software.

## Error Log

The error log has a capacity for 100 entries. Each error is logged by an error number along with the date and time of occurrence. Each error is indexed, with error No. 1 being the least recent error and the nth error being the most recent error. The error log is only accessible through the maintenance mode. When the error log become full, the newest entry replaces the oldest entry.

Instrument Clock

The user interface uses a real-time clock which enables the instrument to maintain an accurate date and time given when the instrument is powered off. Any LCD page which references a time or date also displays the current time and date. The date is displayed in the format "mm/dd/yy". Example : March 12, 1986 is displayed as "09/12/86". The time is displayed in either am/pm format (default) or military (24 hour) format. Am/pm format is "hh:mmxx, where xx is "am" or "pm". Military format is "hhmm hrs." Example 8:48 in the morning is displayed as "8:48 am" or as "0848 hrs". Intervals of time (such as time remaining) are always displayed in the format "xx h yy m" where xx is the number of hours and yy is the number of minutes.

Very Low Power Mode

When the instrument is set up in a certain configuration (e.g. home-health care mode) and is not connected to AC power, the ability of the instrument to operate for extended periods of time becomes even more critical than usual. In such configurations, the instrument will operate in very low power mode. In this mode, the following energy conserving functions are performed:

a) The LCD will blink after two minutes of front panel inactivity. All softkeys and the "more options" key function to re-display the standard page. Pump select switches "A", "B" and "C" as well as "start/stop" and "on/off" function normally.

b) Backlighting is optional; the user has the option of enabling or disabling it. When enabled, the backlighting operates as before (automatically coming on when a control is pressed or when an alarm, advisory, etc. is given); when disabled, the backlighting never comes on. The default for this is to have the backlighting enabled. The user may enable or disable the backlighting from a more option softkey "light", available from the standard page.

**Claims**

1. A user interface for a multimode medication infusion system, characterised by audio generation means (26); a plurality of status indicators (24); an information display (28); a plurality of user inputs (20) ; and a microprocessor (22), operatively connected to, and controlling the audio generation means (26), the status indicators (24), and the information display (28) through the user inputs and through a plurality of input signals from sensors in the infusion system (18).

2. A user interface as claimed in Claim 1, characterised in that the microprocessor is arranged to be connected to a digital computer running specialised software which enables a user to change default values of the operating parameters of the system,

3. A user interface as claimed in Claim 1 or Claim 2, characterised in that the information display (28) is a liquid crystal display.

4. A user interface as claimed in Claim 3, characterised in that the liquid crystal display comprises a screen divided into the following areas: a status line (34) preferably at the top of the screen; a softkey label area (38) preferably at the bottom of the screen; a prompt line (36) preferably adjacent the softkey label area; and an information area (40) preferably between the status and prompt lines.

5. A user interface as claimed in any preceding claim, characterised in that the microprocessor is preprogrammed to provide a plurality of "pages" for display on the information display (28) each "page" determining the function of a subset of the user inputs.

6. A user interface as claimed in Claim 5, characterised in that the "pages" comprise :a standard "page" ; a clinical configuration "page"; an instrument configuration "page"; a maintenance "page"; a pump status "page"; and a total volume infused "page".

7. A user interface as claimed in any preceding claim, characterised in that the user inputs comprise a plurality of hardware switches.

8. A user interface as claimed in Claim 7, characterised in that the hardware switches comprise: an "on/off" power switch; a "standard display" switch; a "start/stop" switch; a "select A" switch for selecting pump A; a "select B" switch for selecting pump B; a "select C" switch for selecting pump C; and a plurality of "softkey" functions that depend on the current operating state of the system and are shown on a "page" displayed on the information display means.

9. A user interface as claimed in Claim 8, characterised in that the hardware switches further comprise a patient-controlled analgesia switch.

10. A user interface as claimed in Claim 8 or Claim 9 characterised in that the "A" switch, "B" switch and "C" switch each have two status indication means associated with them, namely, an "alarm" LED and an "infusing" LED.

*FIG.1*

```
SPECIALIZED
SOFTWARE                    16

        |
        v

COMPUTER                    12

USER          USER              MICROPROCESSOR        22        PUMPS   18
INPUTS   -->  INPUT      -->
              CONTROLS  20                            10

STATUS            AUDIO              DISPLAY
INDICATORS  24    GENERATOR  26      MEANS  28

              USER INTERFACE
```

```
20L   20A        20B              20C              20D

ON          STANDARD         MORE             START
OFF         DISPLAY          OPTIONS          STOP

MINIMED III  INFUSION SYSTEM

30                                                  32

20E        20F              20G              20H

24A  A     24A'  B          24B'  C          24C'
24B        24C              
          20I          20J              20K
```

*FIG.2*

EP 0 319 272 A2

Fig. 3

| 34A | 34B | 34C |
|---|---|---|
| Stopped | Stopped | Stopped |

← STATUS LINE

INFORMATION AREA

— PROMPT LINE —

AAAAAA BBBBBB CCCCCC DDDDDD ← SOFTKEY AREA

38E  38F  38G  38H

34, 40, 36, 38, 32, 42, 44

Fig. 4

NORMAL SIZE TEXT

ABCDEFGHIJKLMNOPQRSTUVWXYZ
0123456789O
abcdefghijklmnopqrstuvwxyz
!@#$%&‡( )−_+=/., ←BLANK CHARACTER
▲▼↑↓→

LARGE SIZE TEXT

0123456789  0123456789
↑
BLANK CHARACTER

Fig. 5

NORMAL TEXT: This is Normal Text

REVERSE TEXT: This is Reverse Text

BLINKING NORMAL TEXT ALTERNATES

BETWEEN THIS: Blinking Text

AND THIS:

BLINKING REVERSE TEXT ALTERNATES

BETWEEN THIS: Blinking Text

AND THIS:

Fig. 6

| Stopped | Stopped | Stopped |
|---|---|---|
| 9 | 99 | 999 |
| ( ) ml/hr | ml/hr | ml/hr |

LOW POWER

Start and Hold affect A

Hold  View  TotVol

| Stopped | Stopped | Alarm |
|---------|---------|-------|
| **9** | **99** | **999** |
| () ml/hr | al/hr | Air in Line |
| VR: 125 | VR: 125 | |
| VI: 57 | VI: 57 | |
| Device Type: General Purpose | | |
| Alarm info: Select C | | |
| Quiet | | |

*Fig. 7*

| Stopped | Stopped | Alarm |
|---------|---------|-------|
Air in Line alarm
Your Options are:
- Remove cassette to clear
- Press Start / Stop to restart
- Press Quiet to silence

Perform one of above or

Quiet

*Fig. 8*

| Stopped | Stopped | Alarm |
|---------|---------|-------|
| **9** | **99** | Air in Line |
| () ml/hr | ml/hr | |

**LOW POWER**

Alarm info: Select C

Quiet

*Fig. 9*

| Instrument Fault | | |
|---------|---------|-------|
| **9** | **99** | **999** |
| () ml/hr | ml/hr | ml/hr |

Memory Failure (135)

Turn instrument off

*Fig. 10*

| Stopped | Stopped | Fault |
|---------|---------|-------|
| **9** | **99** | **999** |
| () ml/hr | ml/hr | Air in Line |
| VR: 125 | VR: 125 | |
| VI: 57 | VI: 57 | |
| Device Type: General Purpose | | |
| Alarm info: Select C | | |
| Quiet | | Reset |

*Fig. 11*

| Stopped | Stopped | Fault |
|---------|---------|-------|
Air in line sensor has failed
failure number 44

Your options are:
- Press Reset to clear
- Press quiet to silence

Perform one of the above or

Quiet          Reset

*Fig. 12*

# EP 0 319 272 A2

**FIG.13**

```
┌──────────────────────────────┐
│ [Stopped] Stopped │         │
│                   │ Service │
│    9      99      │Required │
│                   │   on    │
│ () ml/hr   ml/hr  │  Pump   │
│ VR: 125  VR: 125  │   C     │
│ VI:  57  VI:  57  │         │
├──────────────────────────────┤
│ Device Type: General Purpose │
├──────────────────────────────┤
│ Start and Hold affect A      │
├──────────────────────────────┤
│ [Hold] [View] [TotVol]       │
└──────────────────────────────┘
```

**FIG.14**

```
┌──────────────────────────────┐
│ Stopped │ Stopped │ ████████ │
│                              │
│ Air in line sensor has failed│
│ failure number 44            │
│                              │
│                              │
├──────────────────────────────┤
│ Press Standard Display       │
├──────────────────────────────┤
│ ████ ████ ████ ████          │
└──────────────────────────────┘
```

**FIG.16**

```
                 DISPLAY PUMP
                 INFORMATION
          Y                      DISPLAY PUMP
                                 INFORMATION
‡ SELECTED                 Y              DO NOT DISPLAY
  PUMP ?      CASSETTE                    PUMP INFORMATION
          N   IN PLACE ?           Y
                        N   TIME OUT ?
                                     N    DISPLAY PUMP
                                          INFORMATION

‡ SELECTING ANY PUMP RESETS
  TIME OUT FOR ALL PUMPS WITH
  DISPLAYED INFORMATION
```

**FIG.17**

```
ALARM, ADVISORY              60 SECOND
OR FAULT                     TIME OUT
                                  [A], [B]
            STANDARD DISPLAY              PUMP STATUS
                 PAGE                        PAGE
                                  OR [C]
POWER ON
SEQUENCE       60 SECOND        (STANDARD DISPLAY)
COMPLETE       TIME OUT FROM
               ANY CLINICAL PAGE
```

**FIG.15**

```
┌──────────────────────────────────┐
│ [Stopped] Stopped │ Stopped      │
│                                  │
│    9      99        999          │
│                                  │
│ () ml/hr   ml/hr     ml/hr       │
│ VR: 125  VR: 125   VR: 125       │
│ VI:  57  VI:  57   VI:  57       │
├──────────────────────────────────┤
│ Device Type: General Purpose     │
├──────────────────────────────────┤
│ Start and Hold affect A          │
├──────────────────────────────────┤
│ [Hold] [View] [TotVol]           │
└──────────────────────────────────┘
```

**FIG.18**

```
┌──────────────────────────────────┐
│ [Stopped] Stopped │ Stopped      │
│ [Rate:              999 ml/hr]   │
│ VolRemaining(VR)      ALL  ml    │
│ Time Remaining(TR)    ‡‡‡‡‡       │
│  Vol Infused(VI)      9999 ml    │
│   since 12:34 12/31/87           │
│  Setup: Rate and VR —) Time      │
├──────────────────────────────────┤
│ Press Select to choose line      │
├──────────────────────────────────┤
│ [Select] [▲] [▼] [Clear]         │
└──────────────────────────────────┘
```

Stopped | Stopped | Stopped

TotVol=Total Volume infused
by all three pumps since
TotVol was last cleared
30.0 ml over 12.75 hours
Cleared at: 12/31/87 11:15a
Currently: 01/01/88 12:04a

Press Clear to Reset Volume

| | | | Clear |

*FIG.19*

Clinical Config Page 1 of 4

Time Display format: am/pm

Time: 7:00a
Month: June
Day: 15
Year: 1988

Turn Instrument off to exit

| Select | | | Accept |

*FIG.20*

Clinical Config Page 2 of 4

Device type selection

General Purpose
Operating Room (OR)
Controller Pressure
Neonatal
Home Health Care

Press StdDisp for next page

| Select | | | Accept |

*FIG.21*

Clinical Config Page 3 of 4

Audo Alarm Volume

Audio Volume: lowest

Press StdDisp for next page

| | ▲ | ▼ | Clear |

*FIG.22*

( POWER ON )
( CONFIGURATION )
(ON/OFF) RELEASED &
3 SECOND TIMEOUT

4 PAGES)
CLINICAL CONFIGURATION ←YES— (MORE OPTIONS) HELD —NO→ STANDARD

'CONFIG' MESSAGE FROM PC
'MAINTENANCE' MESSAGE FROM PC

INSTRUMENT CONFIGURATION
MAINTENANCE

A,B OR C | TOTVOL | VIEW | HOLD | MORE

OPTIONS PAGE — PUMP STATUS
VOLUME INFUSED
LIGHT

*FIG.24*

```
┌─────────────────────────────────────┐
│  Clinical Config Page 4 of 4         │
│ ─────────────────────────────────────│
│  Occlusion Alarm:      baseline      │
│                        +5.0 psi      │
│  Max Rate:         999    ml/hr      │
│  Max VR:           9999     ml       │
│  AIL threshold:    100     mcl       │
│  KVO rate:           I    ml/hr      │
│ ─────────────────────────────────────│
│  Turn Instrument off to exit         │
│ ─────────────────────────────────────│
│  ██████  ██████  ██████  ██████      │
└─────────────────────────────────────┘
```

*FIG.23*